# FASCICULE DE BREVET EUROPEEN

(11) **EP 0 732 314 B1**
(45) Date de publication et mention de la délivrance du brevet: **14.03.2001**
(21) Numéro de dépôt: 96200591.4
(22) Date de dépôt: 05.03.1996
(51) Int. Cl.: C07C 17/20, C07C 19/08

(54) **Procédé pour la préparation de difluorométhane**
Verfahren zur Herstellung von Difluormethan
Process for the preparation of difluoromethane

(30) Priorité: 16.03.1995 FR 9503185
(43) Date de publication de la demande: 18.09.1996
(73) Titulaire: SOLVAY (Société Anonyme), 1050 Bruxelles (BE)
(72) Inventeur: Wilmet, Vincent, B-1301 Wavre (BE); Janssens, Francine, B-1800 Vilvoorde (BE)
(74) Mandataire: Jacques, Philippe

(56) Documents cités:
- EP-A- 0 522 639
- US-A- 5 208 395

## Description

La présente invention se rapporte à un procédé pour la préparation de difluorométhane par réaction entre du fluorure d'hydrogène et du dichlorométhane.

Le difluorométhane est un composé de synthèse contenant des atomes de carbone, de fluor et d'hydrogène mais pas de chlore. A ce titre, il peut constituer un substitut des hydrocarbures chlorofluorés entièrement halogénés (CFC) suspectés d'avoir un effet néfaste sur la couche d'ozone. Il s'avère notamment particulièrement intéressant, seul ou en mélange, dans certaines applications en réfrigération et en conditionnement d'air.

Il est connu de longue date que le difluorométhane peut être préparé par hydrofluoration du dichlorométhane en phase liquide, en présence d'halogénures d'antimoine pentavalent (brevets US-A-2,005,711 et US-A-2,749,375). L'halogénure d'antimoine utilisé dans ces procédés connus est toutefois transformé progressivement en halogénure d'antimoine trivalent, inactif en tant que catalyseur de la réaction entre le dichlorométhane et le fluorure d'hydrogène et particulièrement corrosif à l'encontre des matériaux métalliques.

Il est par ailleurs connu, par le brevet US-A-5,208,395, de préparer le difluorométhane par réaction de dichlorométhane avec du fluorure d'hydrogène en phase gazeuse, en présence d'un catalyseur constitué de tétrachlorure d'étain déposé sur du charbon actif. La productivité d'un tel procédé en phase gazeuse, par unité volumique du réacteur, est cependant très faible. En outre, ce procédé connu présente un risque de désactivation du catalyseur.

La présente invention a pour but de fournir un procédé pour la préparation du difluorométhane par réaction entre du fluorure d'hydrogène et du dichlorométhane qui ne présente plus les inconvénients des procédés mentionnés ci-dessus.

L'invention concerne dès lors un procédé pour la préparation du difluorométhane par réaction en phase liquide de dichlorométhane avec du fluorure d'hydrogène, selon lequel on réalise la réaction en présence d'un catalyseur comprenant un halogénure d'un métal choisi parmi le titane et l'étain.

Par halogénure d'un métal choisi parmi le titane et l'étain, on entend désigner un ou plusieurs sels de titane ou d'étain formés à partir d'un ou plusieurs halogénures, tels qu'un halogénure de titane, un halogénure d'étain, un mélange de ces halogénures ou un halogénure mixte de titane et d'étain.

De préférence, le catalyseur comprend au moins 50 % en poids d'halogénure d'un métal choisi parmi le titane et l'étain. Plus préférentiellement encore le catalyseur est essentiellement constitué d'halogénure d'un métal choisi parmi le titane et l'étain.

On retient avantageusement comme halogénure un chlorure ou un fluorure. La mise en oeuvre d'un chlorure est avantageuse en ce que ce composé est économique et facilement disponible. On choisit de préférence un tétrachlorure. La mise en oeuvre d'un fluorure ou d'un chlorofluorure fournit également de bons résultats. Ceux-ci peuvent être obtenus au départ d'un chlorure que l'on soumet à une fluoration au moins partielle. Cette fluoration peut par exemple être réalisée au moyen de fluorure d'hydrogène, préalablement à la mise en contact du catalyseur avec le dichlorométhane. En variante, elle peut être réalisée in situ, au cours de la réaction du dichlorométhane avec le fluorure d'hydrogène.

Les catalyseurs comprenant un halogénure d'étain sont préférés, principalement en raison de leur bonne activité dans le procédé selon l'invention lorsque celui-ci est mis en oeuvre en continu. De très bons résultats ont été obtenus avec du tétrachlorure d'étain comme catalyseur.

Dans le procédé selon l'invention, le catalyseur peut être mis en oeuvre en des quantités variables. Il est généralement mis en oeuvre à raison d'au moins 0,001 mole de catalyseur par mole de dichlorométhane. De préférence, il est mis en oeuvre à raison d'au moins 0,01 mole par mole de dichlorométhane. De très bons résultats ont été obtenus en présence d'au moins environ 0,05 mole de catalyseur par mole de dichlorométhane. En principe, il n'y a pas de limite supérieure à la quantité de catalyseur mise en oeuvre. Par exemple, dans un procédé réalisé en continu, le rapport molaire entre le catalyseur et le dichlorométhane dans la phase liquide peut atteindre 1000. En pratique toutefois, on utilise généralement au maximum 5 moles de catalyseur par mole de dichlorométhane. De préférence, on ne dépasse pas environ 1 mole et plus préférentiellement encore on ne dépasse pas environ 0,5 mole de catalyseur par mole de dichlorométhane.

Le procédé selon l'invention peut être mis en oeuvre de manière continue ou discontinue. Il est entendu que la quantité de catalyseur mise en oeuvre est exprimée, dans un procédé discontinu, par rapport à la quantité initiale de dichlorométhane mise en oeuvre et, dans un procédé continu, par rapport à la quantité stationnaire de dichlorométhane présent dans la phase liquide.

Dans le procédé selon l'invention, le fluorure d'hydrogène et le dichlorométhane sont mis en oeuvre à l'état liquide, dans des rapports molaires variables. Généralement, on met en oeuvre au moins environ 2 moles de fluorure d'hydrogène par mole de dichlorométhane. De préférence, ce rapport est d'au moins environ 4. Le plus souvent, on ne dépasse pas environ 30 moles de fluorure d'hydrogène par mole de dichlorométhane, les valeurs n'excédant pas environ 20 étant spécialement recommandées.

Le procédé selon l'invention peut être réalisé dans de larges gammes de températures et de pressions, dans lesquelles les réactifs sont liquides. Généralement, on travaille à une température d'au moins 75 °C. Une température d'au moins environ 90 °C est préférée. Une température d'au moins environ 100 °C est particulièrement préférée. Le plus souvent, en fonction notamment de la pression admissible, cette température ne dépasse pas 160 °C, les températures inférieures ou égales à environ 140 °C étant spécialement recommandées. Généralement, on travaille à une pression d'au moins 2 bar. Une pression d'au moins environ 5 bar est préférée. Une pression d'au moins environ 10 bar est particulièrement préférée. Le plus souvent, cette pression ne dépasse pas 50 bar, les pressions inférieures ou égales à environ 30 bar étant spécialement recommandées. De manière préférée, on travaille à une température et à une pression auxquelles, en outre, le difluorométhane produit est gazeux.

Le temps de séjour des réactifs dans le réacteur doit être suffisant pour permettre que la réaction du dichlorométhane avec le fluorure d'hydrogène soit réalisée avec un rendement acceptable. Il peut être déterminé aisément en fonction des conditions opératoires retenues. En pratique, le temps de séjour des réactifs dans le réacteur est généralement d'au moins environ 10 minutes. De préférence, il est d'au moins 20 minutes. Le plus souvent, ce temps de séjour ne dépasse pas 6 heures, les valeurs inférieures ou égales à 3 heures étant spécialement recommandées.

Le procédé selon l'invention peut être mis en oeuvre dans tout type de réacteur ou d'appareillage permettant de réunir les conditions décrites et en particulier de résister à la pression et au fluorure d'hydrogène. Le plus souvent, le procédé selon l'invention est réalisé dans un réacteur équipé d'un dispositif de soutirage d'un courant gazeux, par exemple dans un réacteur surmonté d'une colonne et d'un condenseur de reflux. Ce dispositif permet, par un réglage adéquat, de soutirer en phase gazeuse le difluorométhane et le chlorure d'hydrogène produits, tout en maintenant dans le réacteur le dichlorométhane et le fluorure d'hydrogène non transformés, ainsi que, le cas échéant, la majeure partie du chlorofluorométhane qui viendrait à être produit par fluoration partielle du dichlorométhane.

Dans un mode particulier de mise en oeuvre du procédé selon l'invention, on réalise la réaction en présence d'un hydrocarbure halogéné présentant un point d'ébullition d'au moins 25 °C à la pression atmosphérique (autre que le dichlorométhane). Des hydrocarbures halogénés qui conviennent dans ce mode particulier de mise en oeuvre du procédé sont notamment le perchloroéthylène, le trichloroéthylène, le 1,1,2,2,-tétrachloroéthane et le 1,1,1,3,3-pentafluorobutane.

Dans ce mode particulier de mise en oeuvre du procédé selon l'invention, la proportion, dans la phase liquide, de l'hydrocarbure halogéné présentant un point d'ébullition d'au moins 25 °C, est au moins égale à 5 % du poids total de la phase liquide. De préférence, elle est au moins égale à 10 % du poids total de la phase liquide. En général, cette proportion ne dépasse pas 75 % du poids total de la phase liquide. Avantageusement, elle ne dépasse pas 50 % du poids total de la phase liquide.

Les exemples ci-après illustrent l'invention de manière non limitative.

### Exemple 1

Dans un autoclave de 0,5 1 en alliage HASTELLOY B2, équipé d'un agitateur à pales et surmonté d'un condenseur à double enveloppe, on a introduit 0,6 mole de dichlorométhane, 12 moles de fluorure d'hydrogène et 0,06 mole de tétrachlorure de titane. L'autoclave a ensuite été plongé dans un bain thermostatisé, maintenu à une température de 110 °C, et la pression réglée à 15 bar. Le condenseur a été maintenu à une température de 20 °C. Après 5 heures de réaction, 93 % du dichlorométhane mis en oeuvre était transformé, dont 89 % en difluorométhane et 11 % en chlorofluorométhane.

### Exemple 2

L'essai de l'exemple 1 a été répété avec du tétrachlorure d'étain à la place du tétrachlorure de titane. Après 5 heures de réaction, 50 % du dichlorométhane mis en oeuvre était transformé, dont 81 % en difluorométhane et 19 % en chlorofluorométhane.

### Exemple 3

On a introduit dans le réacteur décrit à l'exemple 1 0,20 mol de SnCl₄, 7,50 mol de HF et 1,17 mol de dichlorométhane. Le réacteur a été chauffé à 115 °C sous une pression de 23,5 bar absolu et le condenseur a été maintenu à une température de 45 °C. On a alors alimenté le réacteur en continu à raison de 6,4 g/h de dichlorométhane et de 3 g/h de HF.

Une productivité en difluorométhane en sortie du condenseur de 3,9 g/h a été observée. La réaction a été poursuivie durant 30 heures sans que l'on observe de baisse de la productivité.

### Exemple 4

On a introduit dans le réacteur décrit à l'exemple 1 0,57 mol de SnCl₄, 0,95 mol de 1,1,2,2-tétrachloroéthane et 7,5 mol de HF. Le réacteur a été chauffé à 130 °C sous une pression de 23,5 bar absolu et le condenseur a été maintenu à une température de 45 °C. On a alors alimenté le réacteur en continu à raison de 12 g/h de dichlorométhane et de 8 g/h de HF.

Une productivité en difluorométhane de 6,5 g/h a été observée. La réaction a été poursuivie durant 100 heures sans que l'on observe de baisse de la productivité.

## Revendications

1. Procédé pour la préparation du difluorométhane par réaction en phase liquide de dichlorométhane avec du fluorure d'hydrogène caractérisé en ce qu'on réalise la réaction en présence d'un catalyseur comprenant un halogénure d'un métal choisi parmi le titane et l'étain.

2. Procédé selon la revendication 1, dans lequel le catalyseur est essentiellement constitué d'halogénure d'un métal choisi parmi le titane et l'étain.

3. Procédé selon la revendication 1 ou 2, dans lequel l'halogénure est un chlorure.

4. Procédé selon une quelconque des revendications 1 à 3, dans lequel le catalyseur est le tétrachlorure d'étain.

5. Procédé selon l'une quelconque des revendications 1 à 4, dans lequel le catalyseur est mis en oeuvre en une quantité de 0,001 à 5 moles par mole de dichlorométhane.

6. Procédé selon l'une quelconque des revendications 1 à 5, dans lequel on met en oeuvre de 2 à 30 moles de fluorure d'hydrogène par mole de dichlorométhane.

7. Procédé selon l'une quelconque des revendications 1 à 6, dans lequel on réalise la réaction à une température de 75 à 160 °C.

8. Procédé selon l'une quelconque des revendications 1 à 7, dans lequel on réalise la réaction à une pression de 2 à 50 bar.

9. Procédé selon l'une quelconque des revendications 1 à 8, dans lequel on soutire en phase gazeuse le difluorométhane et le chlorure d'hydrogène produits.

10. Procédé selon l'une quelconque des revendications 1 à 9, dans lequel on réalise la réaction en présence d'un hydrocarbure halogéné présentant un point d'ébullition d'au moins 25 °C à la pression atmosphérique.

## Patentansprüche

1. Verfahren zur Herstellung von Difluormethan durch Reaktion in flüssiger Phase von Dichlormethan mit Fluorwasserstoff, dadurch gekennzeichnet, daß die Reaktion in Gegenwart eines Katalysators durchgeführt wird, umfassend ein Halogenid eines Metalls, ausgewählt aus Titan und Zinn.

2. Verfahren gemäß Anspruch 1, worin der Katalysator im wesentlichen aus einem Halogenid eines Metalls besteht, ausgewählt aus Titan und Zinn.

3. Verfahren gemäß Anspruch 1 oder 2, worin das Halogenid ein Chlorid ist.

4. Verfahren gemäß einem der Ansprüche 1 bis 3, worin der KatalysatorZinntetrachlorid ist.

5. Verfahren gemäß einem der Ansprüche 1 bis 4, worin der Katalysator in einer Menge von 0,001 bis 5 mol pro mol Dichlormethan verwendet wird.

6. Verfahren gemäß einem der Ansprüche 1 bis 5, worin 2 bis 30 mol Fluorwasserstoff pro mol Dichlormethan verwendet werden.

7. Verfahren gemäß einem der Ansprüche 1 bis 6, worin die Reaktion bei einer Temperatur von 75 bis 160°C durchgeführt wird.

8. Verfahren gemäß einem der Ansprüche 1 bis 7, worin die Reaktion bei einem Druck von 2 bis 50 bar durchgeführt wird.

9. Verfahren nach einem der Ansprüche 1 bis 8, bei dem die Produkte Difluormethan und Chlorwasserstoff in Gasphase entnommen werden.

10. Verfahren nach einem der Ansprüche 1 bis 9, worin die Reaktion in Gegenwart eines halogenierten Kohlenwasserstoffs durchgeführt wird, der einen Siedepunkt von mindestens 25°C bei Atmosphärendruck aufweist.

## Claims

1. Process for the preparation of difluoromethane by reaction, in the liquid phase, of dichloromethane with hydrogen fluoride, characterized in that the reaction is performed in the presence of a catalyst comprising a halide of a metal chosen from titanium and tin.

2. Process according to Claim 1, in which the catalyst consists essentially of halide of a metal chosen from titanium and tin.

3. Process according to Claim 1 or 2, in which the halide is a chloride.

4. Process according to any one of Claims 1 to 3, in which the catalyst is tin tetrachloride.

5. Process according to any one of Claims 1 to 4, in which the catalyst is used in an amount of 0.001 to 5 mol per mole of dichloromethane.

6. Process according to any one of Claims 1 to 5, in which from 2 to 30 mol of hydrogen fluoride are used per mole of dichloromethane.

7. Process according to any one of Claims 1 to 6, in which the reaction is performed at a temperature of 75 to 160°C.

8. Process according to any one of 1 to 7, in which the reaction is performed at a pressure of about 2 to 50 bar.

9. Process according to any one of Claims 1 to 8, in which the difluoromethane and the hydrogen chloride produced are withdrawn as a gaseous phase.

10. Process according to any one of Claims 1 to 9, in which the reaction is performed in the presence of a halogenated hydrocarbon having a boiling point of at least 25°C at atmospheric pressure.
